# EUROPEAN PATENT SPECIFICATION

(11) **EP 2 839 781 B1**
(45) Date of publication and mention of the grant of the patent: **27.04.2016**
(21) Application number: 14180799.0
(22) Date of filing: 13.08.2014
(51) Int. Cl.: A61B 5/15, A61B 5/151

(54) **Shock-absorbable lancing device**
Stoßdämpfende Lanzettenvorrichtung
Dispositif autopiqueur absorbant les chocs

(30) Priority: 19.08.2013 TW 102129706; 07.11.2013 TW 102140524
(43) Date of publication of application: 25.02.2015
(73) Proprietor: Biotest Medical Corporation, Taichung City 427 (TW)
(72) Inventor: Cheng, Chen-Heng, 427 Taichung City (TW); CHU, Chun-Feng, 427 Taichung City 427 (TW)
(74) Representative: Becker Kurig Straus

(56) References cited:
- EP-A1- 1 810 616
- EP-A1- 2 617 357
- WO-A2-2005/001418
- WO-A2-2007/005493

## Description

### BACKGROUND OF THE INVENTION

### 1. Field of the Invention

The present invention relates generally to a human blood-sampling device and more particularly, to a shock-absorbable lancing device capable of relieving pain of a testee while penetrating into and exiting a human body.

### 2. Description of the Related Art

When a diabetic or chronic patient needs a blood test, the medical personnel usually use a lancet of a lancing device to penetrate through a testee's skin to make the testee's bleed a little and the little blood is collected for test. The conventional lancing device usually includes a pen-shaped main body, a replaceable lancet, and detachable front cover for conveniently mounting the lancet. The lancet includes a needle mounted a movable mechanism located inside the main body. When the movable mechanism moves to a predetermined position, the needle can exsert out of the front cover to penetrate through the testee's skin into a certain depth and then return.

WO 2007/005493 A2 discloses a lancing device that comprises a main housing, a moveable housing and one or more contacting members, wherein the lancing mechanism is adapted to move between resting, cocking and puncture positions.

In terms of the testee, the quicker the blood sampling process is, the less psychological burden and terror the testee will have. Thus, a user can use a pushrod of the conventional lancet to fire the needle assembly, so the needle assembly can move quickly to exsert out of the front cover of the main body. However, when the lancet penetrates through the testee's skin reach the deepmost point, the lancet collides with the positioning member of the main body to result in great vibration which can make the lancet instantaneously exit the testee's skin the testee, so the testee feels very painful in the process of the blood sampling and the wound made by the lancet becomes larger. Therefore, the conventional lancing device needs further improvement.

### SUMMARY OF THE INVENTION

The primary objective of the present invention is to provide a shock-absorbable lancing device which can absorb the vibration generated, while the lancet enters and exits the human skin and instantaneously returns, to effectively relieve the testee's pain and prevent the testee's wound made by the lancet from expansion.

The foregoing objective of the present invention is attained by the lancing device according to independent claims 1, 5, 7, and 14 and advantageous embodiments are disclosed in the dependent claims. The lancing device is formed of a main body and a front shell mounted to a front end of the main body. The front shell is formed of a housing and a hollow positioning member sleeved into the housing for movably mounting a lancet into the positioning member. The positioning member further includes a shock-absorption layer arranged around an imaginary central axis of the lancet and made of a soft material.

When the lancet is used for blood sampling, the kinetic energy of the lancet can be transmitted to the positioning member and meanwhile, the shock-absorption layer can serve as a shock absorber for providing proper resilience. Taking advantage of the damping effect of the shock-absorption layer, the present invention greatly reduces the vibration generated in the process of movement and instantaneous return of the lancet to make the testee feel hardly painful.

### BRIEF DESCRIPTION OF THE DRAWINGS

FIG. 1 is a side view of a first preferred embodiment of the present invention.
FIG. 2 is a partially sectional view of the first preferred embodiment of the present invention.
FIG. 3A is a partially sectional view of the first preferred embodiment of the present invention, illustrating that the needle of the lancet exserts out of the main body.
FIG. 3B is an enlarged view of a part of FIG. 3B.
FIG. 4 is a partially sectional view of a second preferred embodiment of the present invention.
FIG. 5 is a sectional view of a part of the second preferred embodiment of the present invention.
FIG. 6 is a partially sectional view of a third preferred embodiment of the present invention, illustrating that the needle of the lancet exserts out of the main body.
FIG. 7 is a sectional view of a part of a fourth preferred embodiment of the present invention.
FIG. 8 is a sectional view of a part of a fifth preferred embodiment of the present invention.
FIG. 9 is a sectional view of a part of a sixth preferred embodiment of the present invention.
FIG. 10 is a sectional view of a part of a seventh preferred embodiment of the present invention.

### DETAILED DESCRIPTION OF PREFERRED EMBODIMENTS

Structural features and desired effects of the present invention will become more fully understood by reference to seven preferred embodiments given hereunder. However, it is to be understood that these embodiments are given by way of illustration only, thus are not limitative of the claim scope of the present invention.

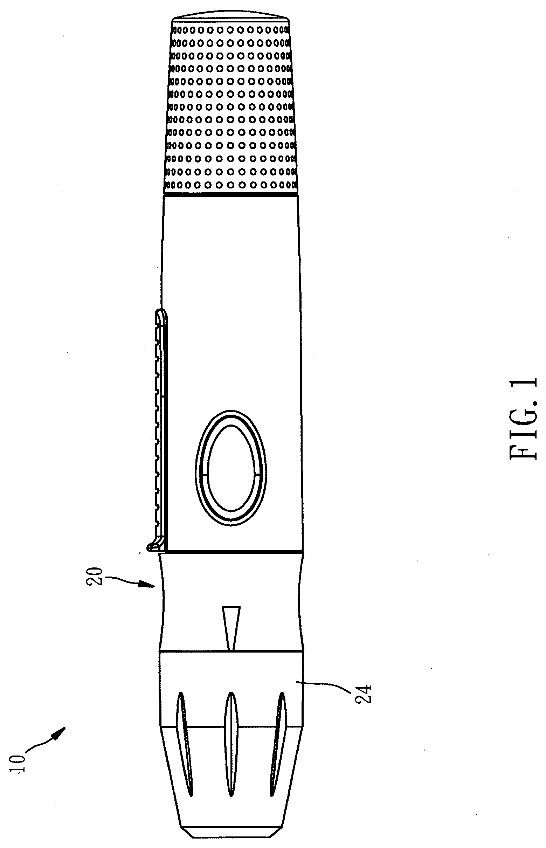

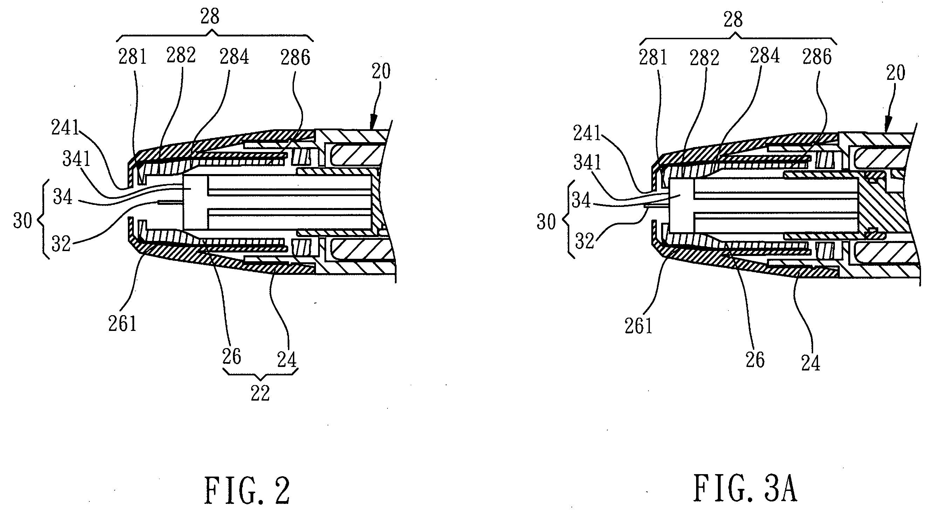

Referring to FIGS. 1-3B, a lancing device 10 constructed according to a first preferred embodiment of the present invention is formed of a main body 20 and a lancet 30. It is worth mentioning that the directions mentioned hereunder are defined according to the recognition of the common people. For example, the lancet 30 exserts out of the front side of the main body 20. The detailed descriptions and operations of these elements as well as their interrelations are recited in the respective paragraphs as follows.

The main body 20 is pen-shaped and includes a detachable front shell 22 mounted to a front end thereof. Referring to FIG. 2 again, the front shell 22 includes a housing 24 and a positioning member 26 mounted inside the housing 24. An outlet 241 is formed at a front end of the housing 24. A slide channel 28 is formed inside the positioning member 26 and communicates with the outlet 241. The outlet 241 and the slide channel 28 are arranged coaxially. Specifically, the slide channel 28 is provided with a circular cross-section and defines a stop portion 281, a small-diameter portion 282, a taper-shaped portion 284, and a large-diameter portion 286, which are arranged in order away from the outlet 241. The small-diameter portion 282 is smaller in diameter than the large-diameter portion 286 and the stop portion 282 is smaller in diameter than the small-diameter portion 282.

The lancet 30 is replaceable for the user and includes a needle 32 and a needle holder 34. The needle holder 34 is slidably received in the slide channel 28 and can be driven by a firing unit (not shown) built in the main body 20 to move forwards and backwards. Since the definite structure of the firing unit is not a key point of the present invention, more recitation thereof is omitted.

The positioning member 26 includes a shock-absorption layer 261 arranged around an imaginary central axis of the lancet 30. In this preferred embodiment, the shock-absorption layer 261 is a hollow resilient member made of rubber ("TPR") or a thermoplastic material and surrounds and covers an external periphery of the positioning member 26 to make the positioning member 26 fit into the housing 24 and to make the lancet 30 inside the positioning member 26 be effectively positioned; in addition, while the lancet 30 is moving, an equable and balanced shock-absorption effect occurs. It is worth mentioning that the aforesaid hollow resilient member can be integrally formed on the positioning member 26 by injection molding.

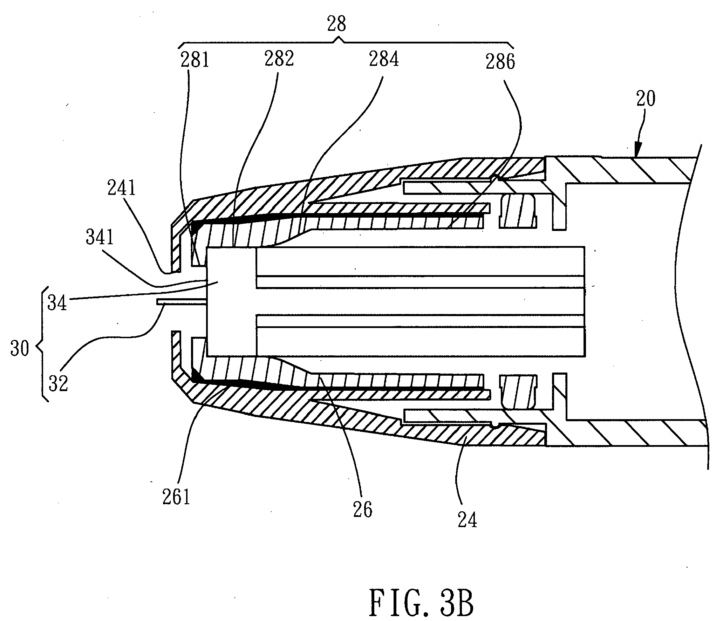

While operating the lancing device 10 of the present invention, referring to FIG. 2 and FIGS. 3A and 3B, the user can make the firing unit drive the lancet 30 to move toward the outlet 241. In the process of the movement of the lancet 30 toward the outlet 241, a front end portion 341 of the needle holder 34 of the lancet 30 can pass through the large-diameter portion 286 and be guided by the taper-shaped portion 284 to enter the small-diameter portion 282; next, the lancet 30 continues to move toward the outlet 241 along the small-diameter portion 282 axially; finally, the front end portion 341 is stopped against the stop portion 281; meanwhile, the needle 32 exserts out of the outlet 241 to further penetrate through the testee's skin and reach the deepmost place of the testee's skin. During the aforesaid movement of the lancet 30, the needle holder 34 is guided by the slide channel 28, so the kinetic energy and radial vibration of the lancet 30 are transmitted to the positioning member 26 and finally absorbed by the shock-absorption layer 261. When the lancet 30 moves forward to make the front end portion 341 contact the stop portion 281, the positioning member 26 stops the lancet 30 from moving forward to lead to a great collision and due to the acting force and the reacting force, the positioning member 26 and the lancet 30 jointly generate great energy of the vibration. Such energy can be absorbed by the shock-absorption layer 261 to make the vibration of the lancet 30 become less to secure smooth movement of the lancet 30, thus greatly lessening the user's pain to make the user feel hardly painful.

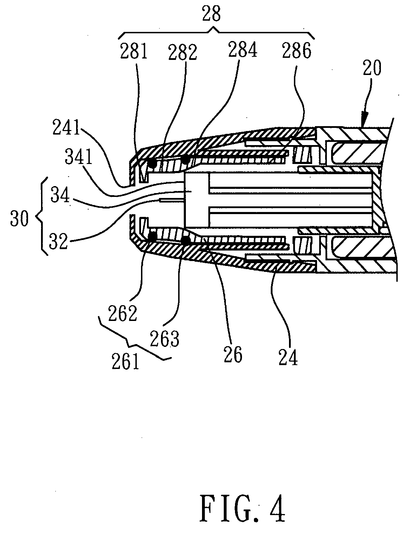

Referring to FIG. 4, a lancing device 10 constructed according to a second preferred embodiment of the present invention is structurally similar to that of the first preferred embodiment, having the following difference. The shock-absorption layer 261 of the second preferred embodiment is formed of two rubber rings 262 and 263, which are sleeved onto a front part and a rear part of the external periphery of the positioning member 26 and spaced from each other, respectively, so excellent shock absorption can be provided likewise.

When the needle holder 34 moves to the small-diameter portion 282, the small-diameter portion 282 includes a cross-section almost matching that of the needle holder 34 in size and meanwhile, the needle 32 of the lancet 32 is going to contact the human skin, so the vibration tends to happen. In terms of the person of skill in the art, the number of the aforesaid rubber rings 262 and 263 can be reduced to become one only and when the one rubber ring is mounted to the front part of the external periphery of the positioning member 26, the same shock absorption can be exploited to make the needle 32 smoothly exsert out of the outlet 241 for less vibration due to the collision and less pain for the user.

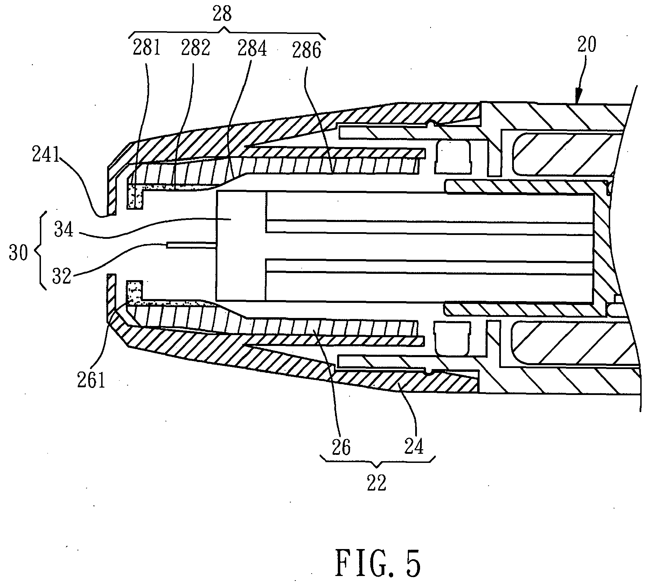

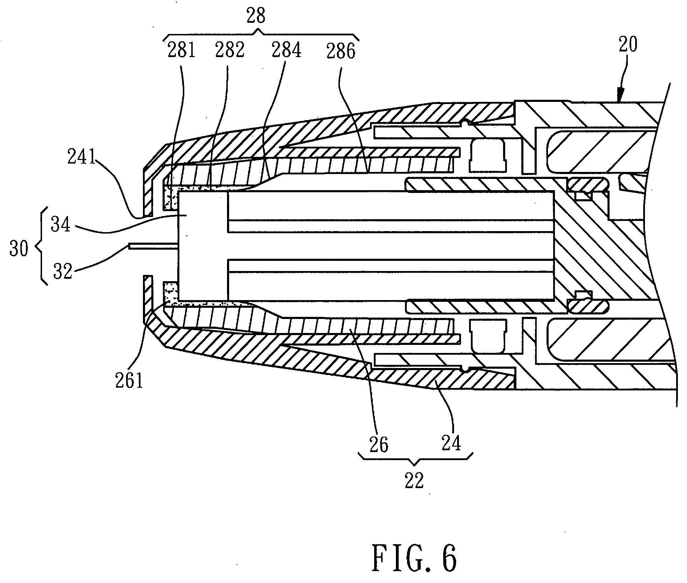

Referring to FIGS. 5 and 6, in a third preferred embodiment of the present invention, the shock-absorption layer 261 is mounted to a front side of an internal periphery of the positioning member 26, namely covering respective undersides of the stop portion 281 and the small-diameter portion 282. In the process of the movement of the lancet 30, the energy generated by the radial vibration of the lancet 30 is likewise transmitted to the shock-absorption layer 261 for its absorption to greatly lessen the vibration of the lancet 30. It is worth mentioning that the shock-absorption layer 261 can be integrally formed on the positioning member 26 by injection molding.

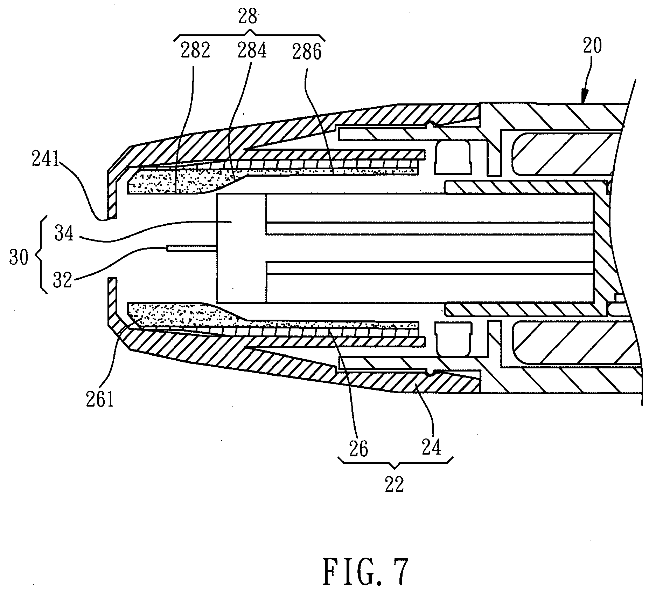

Referring to FIG. 7, a lancing device 10 constructed according to a fourth preferred embodiment of the present invention is structurally similar to that of the third preferred embodiment of the present invention, having the following difference. The shock-absorption layer 261 of the fourth preferred embodiment is completely covered on an internal periphery of the positioning member 26 for providing excellent shock absorption likewise. In addition, the positioning member 26 is provided with another mechanism instead of the stop portion 281 at a front end thereof for controlling the stroke of the lancet 30.

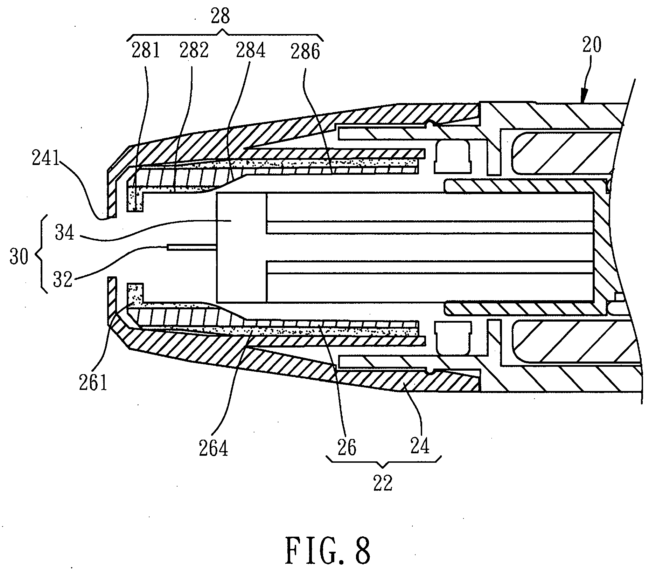

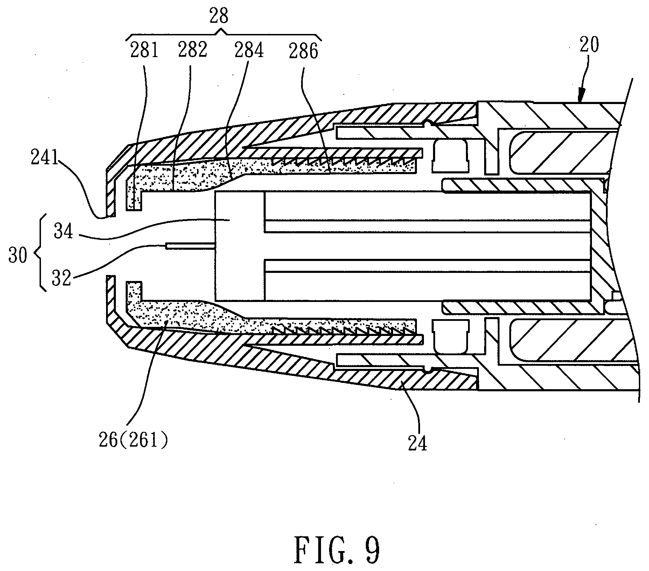

Referring to FIG. 8, a lancing device 10 constructed according to a fifth preferred embodiment of the present invention is structurally similar to that of the third preferred embodiment of the present invention, having the following difference. The positioning member 26 of the fifth preferred embodiment of the present invention includes another shock-absorption layer 264 located at the external periphery thereof for more shock absorption. The person of skill in the art can make the positioning member 26 be made of a shock-absorption soft material. For example, referring to FIG.9, in a sixth preferred embodiment of the present invention, the positioning member 26 can provide shock absorption. It is worth mentioning that the external periphery of the positioning member 26 is equivalent to the shock-absorption layer 261 and can be serrate or non-planar for providing more resilience, or an element made of other material can be embedded into the positioning member 26 to be neither too hard nor too soft for shock absorption.

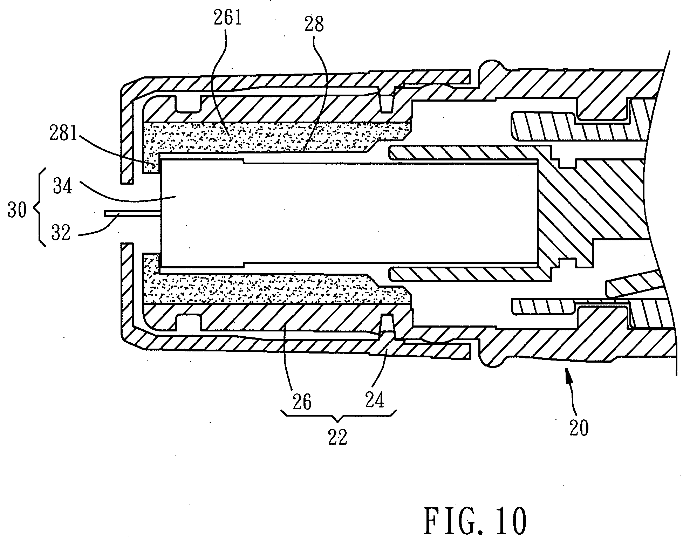

Referring to FIG. 10, in a seventh preferred embodiment of the present invention, which is structurally similar to the fourth preferred embodiment, the slide channel 28 of the positioning member 26 is perfectly straight from a front end thereof to a rear end thereof except that the stop portion 281 remains at the front end of the slide channel 28.

In conclusion, the shock-absorption layer 261 of the present invention can absorb the mechanical energy generated by the rapid movement and collision of the lancet 30 to greatly reduce the vibration of the lancet 30 inside the human skin. The person of skill in the art can selectively adopt the structure and material of the shock-absorption layer 261, as it depends actually, to adjust the damping value. For example, the shock-absorption layer 261 can be formed of a plurality of resilient bars arranged annularly to provide the same effect. Therefore, the present invention indeed can get rid of the pain that the testee fears to facilitate the blood sampling.

## Claims

1. A shock-absorbable lancing device (10) comprising a main body (20) and a front shell (22) mounted to a front end of the main body (20), the front shell (22) being formed of a housing (24) and a positioning member (26) mounted inside the housing (24) for movably receiving a lancet (30), the shock-absorbable lancing device (10) being **characterized in that**:
the positioning member (26) further includes at least one shock-absorption layer (261, 262, 263, 264) arranged around an imaginary central axis of the lancet (30) and made of a soft material, and
wherein the at least one shock-absorption layer (261, 262, 263, 264) is mounted between the positioning member (26) and the housing (24).

2. The shock-absorbable lancing device (10) as defined in claim 1, wherein the at least one shock-absorption layer (261, 264) surrounds and covers an external periphery of the positioning member (26).

3. The shock-absorbable lancing device (10) as defined in claim 2, wherein the at least one shock-absorption layer (261, 264) is a hollow resilient member.

4. The shock-absorbable lancing device (10) as defined in claim 3, wherein the hollow resilient member is integrally formed on the positioning member (26).

5. A shock-absorbable lancing device (10) comprising a main body (20) and a front shell (22) mounted to a front end of the main body (20), the front shell (22) being formed of a housing (24) and a positioning member (26) mounted inside the housing (24) for movably receiving a lancet (30), the shock-absorbable lancing device (10) being **characterized in that**:
the positioning member (26) further includes at least one shock-absorption layer (261, 262, 263, 264) arranged around an imaginary central axis of the lancet (30) and made of a soft material; wherein the at least one shock-absorption layer (262, 263) is at least one rubber ring being hollow resilient and mounted to a front part of the external periphery of the positioning member (26) along a direction that the lancet (30) moves forward.

6. The shock-absorbable lancing device (10) as defined in claim 5, wherein the at least one rubber ring is two in number, the two rubber rings being spaced from each other and mounted to the front part and a rear part of the external periphery of the positioning member (26), respectively, along the direction that the lancet (30) moves forward.

7. A shock-absorbable lancing device (10) comprising a main body (20) and a front shell (22) mounted to a front end of the main body (20), the front shell (22) being formed of a housing (24) and a positioning member (26) mounted inside the housing (24) for movably receiving a lancet (30), the shock-absorbable lancing device (10) being **characterized in that**:
the positioning member (26) further includes at least one shock-absorption layer (261, 262, 263, 264) arranged around an imaginary central axis of the lancet (30) and made of a soft material;
wherein at least one shock-absorption layer (261) is hollow resilient and mounted to an internal periphery of the positioning member (26).

8. The shock-absorbable lancing device (10) as defined in claim 7, wherein at least one shock-absorption layer (261) is a hollow resilient member.

9. The shock-absorbable lancing device (10) as defined in claim 8, wherein at least one shock-absorption layer (261) is integrally formed on the positioning member (26).

10. The shock-absorbable lancing device (10) as defined in claim 7, wherein the positioning member (26) further comprises a shock-absorption layer (264) mounted to an external periphery of the positioning member (26).

11. The shock-absorbable lancing device (10) as defined in claim 7, wherein the positioning member (26) is made of a material identical to that of the at least one shock-absorption layer (261, 264).

12. The shock-absorbable lancing device (10) as defined in claim 1, wherein at least one shock-absorption layer (261, 264) is made of a thermoplastic material.

13. The shock-absorbable lancing device (10) as defined in claim 1, wherein the housing (24) and the positioning member (26) are arranged coaxially, the housing (24) having an outlet (241), the positioning member (26) having a slide channel (28) formed therein for communication with the outlet (241), the slide channel (28) defining a small-diameter portion (282), a taper-shaped portion (284), and a large-diameter (286) in order away from the outlet (241).

14. A shock-absorbable lancing device (10) comprising a main body (20) and a front shell (22) mounted to a front end of the main body (20), the front shell (22) being formed of a housing (24) and a positioning member (26) mounted inside the housing (24) for movably receiving a lancet (30), the shock-absorbable lancing device (10) being **characterized in that**:
the positioning member (26) further includes at least one shock-absorption layer (261, 262, 263, 264) arranged around an imaginary central axis of the lancet (30) and made of a soft material;
the at least one shock-absorption layer (261, 264) is hollow resilient, extends axially along the small-diameter portion (282), and is mounted between the positioning member (26) and the housing (24).

## Patentansprüche

1. Stoßdämpfende Lanzettenvorrichtung (10) umfassend einen Hauptkörper (20) und eine vordere Hülse (22), die an einem Vorderende des Hauptkörpers (20) angeordnet ist, wobei die vordere Hülse (22) aus einem Gehäuse (24) und einem Positionierungselement (26) gebildet ist, das in dem Gehäuse (24) angeordnet ist, um eine Lanzette (30) beweglich aufzunehmen, die stoßdämpfende Lanzettenvorrichtung (10) ist **dadurch gekennzeichnet, dass**:
das Positionierungselement (26) ferner wenigstens eine stoßdämpfende Schicht (261, 262, 263, 264) beinhaltet, die um eine imaginäre mittige Achse der Lanzette (30) herum angeordnet und aus einem weichen Material hergestellt ist, und
die wenigstens eine stoßdämpfende Schicht (261, 262, 263, 264) zwischen dem Positionierungselement (26) und dem Gehäuse (24) angeordnet ist.

2. Stoßdämpfende Lanzettenvorrichtung (10) nach Anspruch 1, worin die wenigstens eine stoßdämpfende Schicht (261, 264) einen äußeren Umfang des Positionierungselements (26) umgibt und bedeckt.

3. Stoßdämpfende Lanzettenvorrichtung (10) nach Anspruch 2, worin die wenigstens eine stoßdämpfende Schicht (261, 264) ein hohles, federndes Element ist.

4. Stoßdämpfende Lanzettenvorrichtung (10) nach Anspruch 3, worin das hohle, federnde Element einstückig auf dem Positionierungselements (26) gebildet ist

5. Stoßdämpfende Lanzettenvorrichtung (10) umfassend einen Hauptkörper (20) und eine vordere Hülse (22), die an einem Vorderende des Hauptkörpers (20) angeordnet ist, wobei die vordere Hülse (22) aus einem Gehäuse (24) und einem Positionierungselement (26) gebildet ist, das in dem Gehäuse (24) angeordnet ist, um eine Lanzette (30) beweglich aufzunehmen, die stoßdämpfende Lanzettenvorrichtung (10) ist **dadurch gekennzeichnet, dass**:
das Positionierungselement (26) ferner wenigstens eine stoßdämpfende Schicht (261, 262, 263, 264) beinhaltet, die um eine imaginäre mittige Achse der Lanzette (30) herum angeordnet und aus einem weichen Material hergestellt ist, und
die wenigstens eine stoßdämpfende Schicht (262, 263) wenigstens ein Gummiring ist, der hohl und federnd ist und an einem Vorderteil des äußeren Umfangs des Positionierungselements (26) entlang einer Richtung gebildet ist, in die sich die Lanzette (30) nach vorne bewegt.

6. Stoßdämpfende Lanzettenvorrichtung (10) nach Anspruch 5, worin der wenigstens eine Gummiring zwei Gummiringe ist und die zwei Gummiringe voneinander beabstandet und an dem Vorderteil beziehungsweise einem hinteren Teil des äußeren Umfangs des Positionierungselements (26) entlang einer Richtung angeordnet sind, in die sich die Lanzette (30) nach vorne bewegt.

7. Stoßdämpfende Lanzettenvorrichtung (10) umfassend einen Hauptkörper (20) und eine vordere Hülse (22), die an einem Vorderende des Hauptkörpers (20) angeordnet ist, wobei die vordere Hülse (22) aus einem Gehäuse (24) und einem Positionierungselement (26) gebildet ist, das in dem Gehäuse (24) angeordnet ist, um eine Lanzette (30) beweglich aufzunehmen, die stoßdämpfende Lanzettenvorrichtung (10) ist **dadurch gekennzeichnet, dass**:
das Positionierungselement (26) ferner wenigstens eine stoßdämpfende Schicht (261, 262, 263, 264) beinhaltet, die um eine imaginäre mittige Achse der Lanzette (30) herum angeordnet und aus einem weichen Material hergestellt ist, und
die wenigstens eine stoßdämpfende Schicht (261) hohl und federnd und an einem inneren Umfang des Positionierungselements (26) angeordnet ist.

8. Stoßdämpfende Lanzettenvorrichtung (10) nach Anspruch 7, worin wenigstens eine stoßdämpfende Schicht (261) ein hohles, federndes Element ist.

9. Stoßdämpfende Lanzettenvorrichtung (10) nach Anspruch 8, worin wenigstens eine stoßdämpfende Schicht (261) einstückig auf dem Positionierungselement (26) gebildet ist.

10. Stoßdämpfende Lanzettenvorrichtung (10) nach Anspruch 7, worin das Positionierungselement (26) ferner eine stoßdämpfende Schicht (264) umfasst, die an einem äußeren Umfang des Positionierungselements (26) angeordnet ist.

11. Stoßdämpfende Lanzettenvorrichtung (10) nach Anspruch 7, worin das Positionierungselement (26) aus einem Material hergestellt ist, das mit dem der wenigstens einen stoßdämpfenden Schicht (261, 264) identisch ist.

12. Stoßdämpfende Lanzettenvorrichtung (10) nach Anspruch 1, worin wenigstens eine stoßdämpfende Schicht (261, 264) aus einem thermoplastischen Material hergestellt ist.

13. Stoßdämpfende Lanzettenvorrichtung (10) nach Anspruch 1, worin das Gehäuse (24) und das Positionierungselement (26) koaxial angeordnet sind, wobei das Gehäuse (24) einen Auslass (241) und das Positionierungselement (26) einen Gleitkanal (28) aufweist, der darin ausgebildet vorliegt, um mit dem Auslass (241) in Verbindung zu stehen, wobei der Gleitkanal (28) einen Bereich mit geringem Durchmesser (282), einen spitz zulaufenden Bereich (284), sowie einen Bereich mit großem Durchmesser (286) in dieser Reihenfolge weg von dem Auslass (241) definiert.

14. Stoßdämpfende Lanzettenvorrichtung (10) umfassend einen Hauptkörper (20) und eine vordere Hülse (22), die an einem Vorderende des Hauptkörpers (20) angeordnet ist, wobei die vordere Hülse (22) aus einem Gehäuse (24) und einem Positionierungselement (26) gebildet ist, das in dem Gehäuse (24) angeordnet ist, um eine Lanzette (30) beweglich aufzunehmen, die stoßdämpfende Lanzettenvorrichtung (10) ist **dadurch gekennzeichnet, dass**:
das Positionierungselement (26) ferner wenigstens eine stoßdämpfende Schicht (261, 262, 263, 264) beinhaltet, die um eine imaginäre mittige Achse der Lanzette (30) herum angeordnet und aus einem weichen Material hergestellt ist, und
die wenigstens eine stoßdämpfende Schicht (261, 264) hohl und federnd ist und sich axial entlang eines Bereichs mit geringem Durchmesser (282) erstreckt, sowie zwischen dem Positionierungselement (26) und dem Gehäuse (24) angeordnet ist.

## Revendications

1. Dispositif autopiqueur à amortissement (10) comprenant un corps principal (20) et une enveloppe avant (22) montée à une extrémité avant du corps principal (20), l'enveloppe avant (22) étant formée d'un logement (24) et d'un élément de positionnement (26) monté à l'intérieur du logement (24) pour recevoir de manière mobile une lancette (30), le dispositif autopiqueur à amortissement (10) étant **caractérisé en ce que** :
l'élément de positionnement (26) comprend en outre au moins une couche d'amortissement (261, 262, 263, 264) disposée autour d'un axe central imaginaire de la lancette (30) et constituée d'un matériau mou, et
dans lequel ladite au moins une couche d'amortissement (261, 262, 263, 264) est montée entre l'élément de positionnement (26) et le logement (24).

2. Dispositif autopiqueur à amortissement (10) selon la revendication 1, dans lequel ladite au moins une couche d'amortissement (261, 264) entoure et couvre une périphérie externe de l'élément de positionnement (26).

3. Dispositif autopiqueur à amortissement (10) selon la revendication 2, dans lequel ladite au moins une couche d'amortissement (261, 264) est un élément élastique creux.

4. Dispositif autopiqueur à amortissement (10) selon la revendication 3, dans lequel l'élément élastique creux est formé en une seule pièce sur l'élément de positionnement (26).

5. Dispositif autopiqueur à amortissement (10) comprenant un corps principal (20) et une enveloppe avant (22) montée à une extrémité avant du corps principal (20), l'enveloppe avant (22) étant formée d'un logement (24) et d'un élément de positionnement (26) monté à l'intérieur du logement (24) pour recevoir de manière mobile une lancette (30), le dispositif autopiqueur à amortissement (10) étant **caractérisé en ce que** :
l'élément de positionnement (26) comprend en outre au moins une couche d'amortissement (261, 262, 263, 264) disposée autour d'un axe central imaginaire de la lancette (30) et constituée d'un matériau mou ; dans lequel ladite au moins une couche d'amortissement (262, 263) est au moins un anneau en caoutchouc étant creux et élastique, et monté à une partie frontale de la périphérie externe de l'élément de positionnement (26) le long d'une direction vers laquelle avance la lancette (30).

6. Dispositif autopiqueur à amortissement (10) selon la revendication 5, dans lequel ledit au moins un anneau en caoutchouc est au nombre de deux, les deux anneaux en caoutchouc étant espacés l'un de l'autre et montés à la partie frontale et à une partie arrière de la périphérie externe de l'élément de positionnement (26), respectivement, le long de la direction vers laquelle avance la lancette (30).

7. Dispositif autopiqueur à amortissement (10) comprenant un corps principal (20) et une enveloppe avant (22) montée à une extrémité avant du corps principal (20), l'enveloppe avant (22) étant formée d'un logement (24) et d'un élément de positionnement (26) monté à l'intérieur du logement (24) pour recevoir de manière mobile une lancette (30), le dispositif autopiqueur à amortissement (10) étant **caractérisé en ce que** :
l'élément de positionnement (26) comprend en outre au moins une couche d'amortissement (261, 262, 263, 264) disposée autour d'un axe central imaginaire de la lancette (30) et constituée d'un matériau mou ;
dans lequel au moins une couche d'amortissement (261) est creuse et élastique, et montée à une périphérie interne de l'élément de positionnement (26).

8. Dispositif autopiqueur à amortissement (10) selon la revendication 7, dans lequel au moins une couche d'amortissement (261) est un élément élastique creux.

9. Dispositif autopiqueur à amortissement (10) selon la revendication 8, dans lequel au moins une couche d'amortissement (261) est formée en une seule pièce sur l'élément de positionnement (26).

10. Dispositif autopiqueur à amortissement (10) selon la revendication 7, dans lequel l'élément de positionnement (26) comprend en outre une couche d'amortissement (264) montée à une périphérie externe de l'élément de positionnement (26).

11. Dispositif autopiqueur à amortissement (10) selon la revendication 7, dans lequel l'élément de positionnement (26) est constitué d'un matériau identique à celui de ladite au moins une couche d'amortissement (261, 264).

12. Dispositif autopiqueur à amortissement (10) selon la revendication 1, dans lequel au moins une couche d'amortissement (261, 264) est constituée d'un matériau thermoplastique.

13. Dispositif autopiqueur à amortissement (10) selon la revendication 1, dans lequel le logement (24) et l'élément de positionnement (26) sont disposés de manière coaxiale, le logement (24) comportant une sortie (241), l'élément de positionnement (26) comportant un canal de glissement (28) formé en son sein pour la communication avec la sortie (241), le canal de glissement (28) définissant une partie de petit diamètre (282), une partie de forme effilée (284), et un grand diamètre (286) successivement en s'éloignant de la sortie (241).

14. Dispositif autopiqueur à amortissement (10) comprenant un corps principal (20) et une enveloppe avant (22) montée à une extrémité avant du corps principal (20), l'enveloppe avant (22) étant formée d'un logement (24) et d'un élément de positionnement (26) monté à l'intérieur du logement (24) pour recevoir de manière mobile une lancette (30), le dispositif autopiqueur à amortissement (10) étant **caractérisé en ce que** :
l'élément de positionnement (26) comprend en outre au moins une couche d'amortissement (261, 262, 263, 264) disposée autour d'un axe central imaginaire de la lancette (30) et constituée d'un matériau mou ;
ladite au moins une couche d'amortissement (261, 264) est creuse et élastique, s'étend axialement le long de la partie de petit diamètre (282), et est montée entre l'élément de positionnement (26) et le logement (24).
